# Europäisches Patentamt
## European Patent Office
## Office européen des brevets

(11) Veröffentlichungsnummer: **0 279 361 B1**

(12) **EUROPÄISCHE PATENTSCHRIFT**

(45) Veröffentlichungstag der Patentschrift: **13.05.92**

(51) Int. Cl.⁵: **G01L 1/14**, G01L 15/00

(21) Anmeldenummer: **88102035.8**

(22) Anmeldetag: **11.02.88**

(54) **Vorrichtung zur Messung der flächigen Verteilung von Druckkräften.**

(30) Priorität: **16.02.87 DE 3704870**

(43) Veröffentlichungstag der Anmeldung:
**24.08.88 Patentblatt 88/34**

(45) Bekanntmachung des Hinweises auf die
Patenterteilung:
**13.05.92 Patentblatt 92/20**

(84) Benannte Vertragsstaaten:
**DE FR GB IT**

(56) Entgegenhaltungen:
**EP-A- 0 172 784**
**DE-A- 3 025 362**
**DE-A- 3 411 528**
**US-A- 4 262 532**

(73) Patentinhaber: **Seitz, Peter**
**Möhlstrasse 29**
**W-8000 München 80(DE)**

(72) Erfinder: **Seitz, Peter**
**Möhlstrasse 29**
**W-8000 München 80(DE)**

(74) Vertreter: **Bohnenberger, Johannes, Dr. et al**
**Meissner, Bolte & Partner Widenmayerstrasse 48 Postfach 86 06 24**
**W-8000 München 86(DE)**

**Beschreibung**

Die Erfindung betrifft eine Vorrichtung zur Messung der flächigen Verteilung von Druckkräften nach dem Oberbegriff des Patentanspruchs 1 oder 7.

Aus der DE-OS 24 48 398 und der DE-OS 28 00 844 sind Meßvorrichtungen bekannt, bei denen elastische Matten mit Kondensatorflächen vorgesehen sind, um die auf die Matten wirkenden Normalkräfte messen zu können. Eine flächige Verteilung der Druckkräfte kann jedoch mit diesen Vorrichtungen nicht aufgelöst werden.

Aus der DE-OS 25 29 475 ist eine Meßanordnung zur flächigen Verteilung von Druckkräften bekannt, auf die hier besonders Bezug genommen wird. Die erfindungsgemäße Matte kann mit der dort gezeigten Auswertelektronik betrieben werden, so daß auf die Auswertelektronik im weiteren nicht näher eingegangen wird.

Aufbauend auf der vorgenannten DE-OS 25 29 475 ist mit der DE-PS 30 25 362 eine Vorrichtung der eingangs genannten Art bekannt geworden, bei der eine besondere Konzeption der Leiterbahnen aufgezeigt wird. Diese Konzeption wurde im Hinblick darauf gewählt, daß einfache Leiterbahnen, die auf einem elastisch verformbaren, flächigen Dielektrikum befestigt sind, angeblich schlechte elektrische Eigenschaften (Nichtlinearität, Hysterese) und auch schlechte mechanische Eigenschaften (Relaxationserscheinungen, Materialermüdung, geringe Flexibilität) mit sich bringen, wobei weiterhin angeblich eine ausschließliche Verwendbarkeit auf hartem, ebenem Untergrund gegeben sei. Weiterhin wird in dieser Veröffentlichung darauf hingewiesen, daß derartige einfache Anordnungen ein mechanisches Überkoppeln auf benachbarte Kondensatoren mit sich bringen. Um diese Nachteile zu vermeiden, wird in der Druckschrift vorgeschlagen, besondere Gitter-Leiterbahnen in einer besonderen Art anzubringen. Diese Anordnung ist jedoch außerordentlich kompliziert und hinsichtlich Flexibilität dann besonders schlecht geeignet, wenn z.B. eine in einem Schuh zu tragende Meßanordnung geschaffen werden soll. Auch bei Verwendung der Meßfläche als Auflage für einen Kfz-Sitz (um das Sitzverhalten von Probanden zu messen) treten Probleme hinsichtlich Flexibilität auf. Weiterhin ist eine Massenfertigung einer solchen Meßmatte aufgrund des großen betriebenen Aufwandes nahezu unmöglich.

Aus der DE-OS 34 11 528 ist ebenfalls eine Meßanordnung der eingangs genannten Art bekannt, die ebenfalls auf eine Verbesserung der elektrischen und mechanischen Eigenschaften hinzielt. Auch in dieser Druckschrift wird darauf hingewiesen, daß metallisierte Kunststoff-Folienstreifen als Leiterbahnen nicht geeignet seien. In der Druckschrift wird vorgeschlagen, die einzelnen Meßflächen, die sich an den Kreuzungspunkten der Leiterbahnen ergeben, durch Einschnitte voneinander zu trennen, um so eine mechanische Entkopplung zu erzielen. Auch dieser Vorschlag bedingt jedoch hohe Fertigungskosten und führt darüber hinaus zu einer hohen Bruchempfindlichkeit der Anordnung.

Aus der EP-A2-17 27 84 ist eine Vorrichtung nach dem Oberbegriff des Patentanspruches 1 oder 7 bekannt. An keiner Stelle der Druckschrift wird jedoch erläutert, wie man die dort vorgesehen Metallstreifen kontaktieren soll.

Der Erfindung liegt die Aufgabe zugrunde, eine Vorrichtung der eingangs genannten Art dahingehend weiterzubilden, daß bei geringem Aufwand eine präzise und dennoch robuste Meßanordnung hoher Flexibilität erzielbar ist.

Diese Aufgabe wird entweder dadurch gelöst, daß das elastisch verformbare Dielektrikum als flächiges Dielektrikum aus Kunststoff ausgebildet ist, und daß die Kondensatorelemente zusammen mit den Leitungen auf Trägerfolien aus Kunststoff als Träger aufgedruckt sind. Alternativ wird diese Aufgabe dadurch gelöst, daß das elastisch verformbare Dielektrikum als flächiges Dielektrikum aus Kunststoff ausgebildet ist, und daß die Kondensatorelemente zusammen mit den Leitungen auf das Dielektrikum als Träger aufgedruckt sind.

Überraschenderweise konnte es gezeigt werden, daß entgegen der bisher geltenden Auffassung mit einfachen Leiterbahnen dann gearbeitet werden kann, wenn diese auf Trägerfolien aus Kunststoff aufgedruckt sind. Man muß also keine Gitterstrukturen vorsehen, um eine hinreichende mechanische Entkopplung und gleichzeitig gute elektrische Eigenschaften zu erzielen.

Ein weiterer wesentlicher Punkt der vorliegenden Erfindung liegt darin, daß bei Verwendung von aufgedruckten Leiterbahnen und Abschirmflächen eine besonders günstige Fertigung gegeben ist. Man kann dann bei einer entsprechenden Formgebung, wie sie in den abhängigen Ansprüchen beschrieben ist, auf einfachste Weise besonders störsichere Meßmatten aufbauen, wobei der Aufwand für anfallende Handarbeiten extrem niedrig ist. Die Haltbarkeit der Anordnung ist gegenüber den bisherigen Vorschlägen wesentlich gesteigert.

Die Leitungen bestehen hierbei aus metall- vorzugsweise aber aus silber-gefülltem Kunstharz. Dieses Material weist besonders gute Selbstheil-Eigenschaften auf, so daß bei Beanspruchung auftretende Bruchstellen im Laufe der Zeit von selbst verschwinden.

Bei einer bevorzugten Variante der Erfindung sind die Kondensatorelemente und die dazugehörigen Leitungen auf zwei verschiedenen Seiten der Trägerfolie angebracht, wobei die Verbindung zwischen Leitungen und Kondensatorelementen über Durch-Kontaktierungen vorgenommen sind, wie sie bei Leiterplatten für gedruckte Schaltungen bereits bekannt sind. Die Verbindung zwischen Leitungen und Kondensatorelementen wird also durch Innenauskleidungen von durchgehenden Ausnehmungen in den Trägerfolien gebildet, wobei diese Innenauskleidungen aus demselben Material wie die Kondensatorelemente bzw. wie die Leitungen bestehen.

Wenn eine sehr hohe Auflösung (hohe Anzahl von Kondensatorelementen pro Flächeneinheit) gewünscht ist, so kann es geschehen, daß die Kondensatorelemente zu dicht aneinander gerückt aufgebaut werden müssen. In diesem Fall ist es von Vorteil, wenn man verschiedene Meßmatten-Ebenen vorsieht, wobei jede Ebene Kondensatorelemente mit Abstand voneinander trägt und diese Kondensatorelemente in den verschiedenen Ebenen so gegeneinander versetzt angeordnet sind, daß bei Aufeinanderprojektion aller Kondensatorelemente eine durchgehende Meßfläche entsteht.

Um einen elektrisch besonders störunempfindlichen Aufbau zu erreichen, ist es von Vorteil, wenn man Abschirmflächen vorsieht, welche die Kondensatorelemente und gegebenenfalls weiterhin auch die Leitungen nach außen elektrisch abschirmen. Hierbei ist es von Vorteil, wenn auf der, den Kondensatorelementen gegenüberliegenden Seite auf den Trägerfolien Abschirmflächen aufgedruckt sind, welche die Kondensatorelemente, gegebenenfalls auch die Leitungen überdecken.

Wenn man eine relativ steife Trägerfolie (z.B. aus PVC) vorsieht, so ist es von Vorteil, wenn diese zwischen den Kondensatorelementen mindestens abschnittsweise durchtrennt sind.

Eine wesentliche Einsatzmöglichkeit für die erfindungsgemäße Vorrichtung ist das Messen von Kräften, die auf die Fußsohle beim Gehen, Laufen und Stehen wirken. Wenn man die erfindungsgemäße Vorrichtung in einen Schuh einlegen will, so wird die oben angegebene Aufgabe durch eine Vorrichtung gelöst, bei der die Meßfläche der Form einer Fuß-Auftrittsfläche angepaßt ist und in einen Schuh eines Probanden einlegbar ist, wobei hier die Kondensatorelemente reihenförmig angeordnet sind und ein Satz von Kondensatorelementen quer zur Fußlängsachse, der andere Satz von Kondensatorelementen parallel zur Fußlängsachse verläuft. Die Leitungen sind vorzugsweise im Bereich des Tuberosithas Ossis Navicularis zusammen mit einem Trägerfolienstreifen als Anschlußflachkabel aus dem Schuh herausgeführt. Die so entstehende Anordnung ist sehr leicht und kostengünstig herstellbar aber dennoch sehr haltbar, so daß sie sich zum ständigen Einsatz z.B. in der Klinik oder bei einem Orthopäden eignet.

Weitere Details ergeben sich aus den abhängigen Ansprüchen und der nachfolgenden Beschreibung von Ausführungsbeispielen bevorzugter Ausführungsformen der Erfindung, die anhand von Abbildungen näher beschrieben werden. Hierbei zeigen:

| Fig. 1 und 2 | Trägerfolien mit aufgedruckten Leiterbahnen im Zustand nach dem Bedrucken; |
| Fig. 3 | eine zugeschnittene Trägerfolie mit Leiterbahn, wie sie aus der Folie nach Fig. 1 herstellbar ist; |
| Fig. 4 | einen Schnitt entlang der Linie IV-IV aus Fig. 3; |
| Fig. 5 | eine Anordnung nach Fig. 3 mit umgeklappten Abschirmflächen; |
| Fig. 6 | eine Anordnung nach Fig. 2 im Fertigungsstadium, wie die Anordnung nach Fig. 5; |
| Fig. 7 | einen Schnitt entlang der Linie VII-VII aus Fig. 6; |
| Fig. 8 | einen Schnitt entlang der Linie VIII-VIII aus Fig. 6; |
| Fig. 9 | eine fertige Meßfläche; |
| Fig 10 | einen Schnitt entlang der Linie X-X aus Fig. 9; |
| Fig. 11 | eine Anordnung nach Fig. 9, jedoch unter Fortlassung der in Draufsicht oberen Abschirmflächen. |
| Fig. 12 bis 14 | eine zweite bevorzugte Ausführungsform der Erfindung in Draufsicht (auf unfertige Folien) bzw. in einer Schnittdarstellung (durch eine vollständig zusammengebaute Vorrichtung); und |
| Fig. 15 | eine perspektivische Schemadarstellung einer dritten bevorzugten Ausführungsform der Erfindung. |

Im folgenden wird eine bevorzugte Ausführungsform der Erfindung, die sich auf eine in einen Schuh einlegbare Meßfläche bezieht, näher beschrieben. Aus der folgenden Beschreibung geht auch das Verfahren zur Herstellung der Vorrichtung hervor.

Bei der Herstellung dieser bevorzugten Ausführungsform geht man zunächst von PVC-folien 12 und 13 aus, die auf der einen Fläche mit den als Leitbahnen 10 und 11 ausgebildeten Kondensatorelementen sowie den in diese übergehende, als leitfähige Elemente 14 und 15 ausgebildeten Leitungen und auf der anderen Fläche mit den entsprechenden Abschirmflächen 17 und 18 bedruckt werden. Hierbei ist es überraschend, daß PVC als Grundmaterial für die Trägerfolien 12, 13 verwendbar ist.

Das leitfähige Material, mit dem die Trägerfolie bedruckt wird, ist ein metallgefülltes Kunstharz, bei dem die Metallfüllung vorzugsweise im wesentlichen aus Silber besteht. Vorzugsweise ist hierbei das Silber in Form von mikroskopischen Fädchen vorgesehen, das dazu führt, daß bei einem eventuellen Bruch eine "Selbstheilung" durch einfaches Erwärmen und dadurch eine Neuordnung der "Fädchen" stattfinden kann.

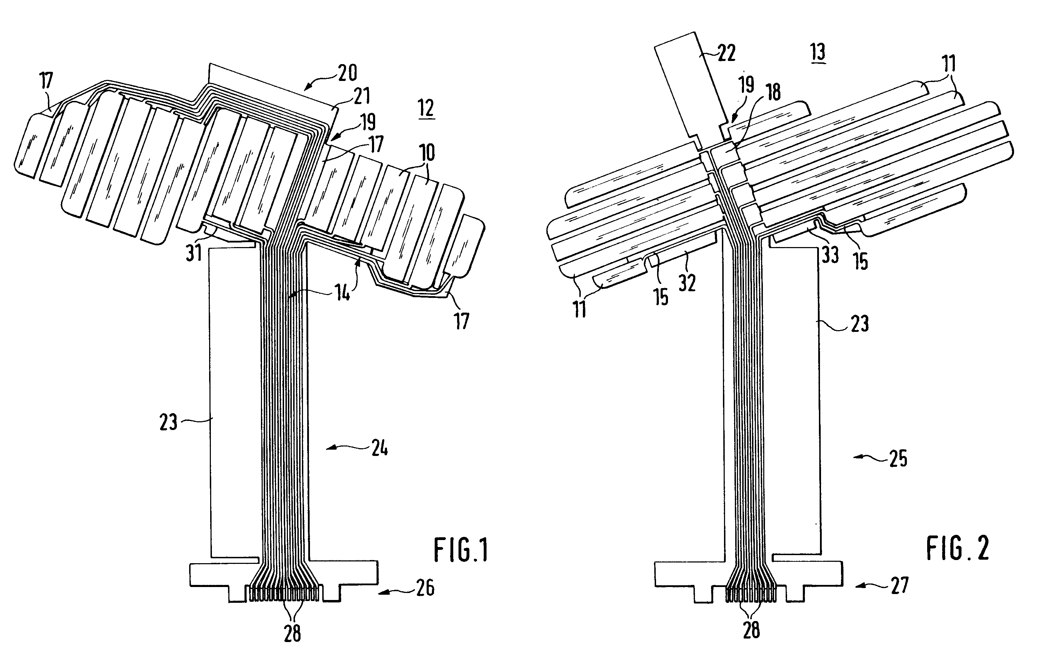

Die in Fig. 1 gezeigte Fläche weist quer zur Fußlängsachse orientierte Leiterbahnen 10 auf, bei der im Bereich zwischen Ferse und Vorderfuß eine der Leiterbahnen fortgelassen ist. In diesem Bereich kann also keine Messung erfolgen, was aber wegen der dort bei normalem Fuß nicht auftretenden Kräfte unerheblich ist.

Die Leiterbahnen 10 sind über leitfähige Elemente 14 kontaktiert. Die leitfähigen Elemente 14 sind von der eigentlichen Meßfläche als Anschlußflachkabel 24 geradlinig fortgeführt. Die Fortführung geschieht hierbei in einem Winkel schräg nach hinten in Richtung auf die Ferse, was sich als besonders vorteilhaft erwiesen hat, wie dies weiter unten noch näher beschrieben wird.

Der Fersenbereich der Anordnung wird über leitfähige Elemente 14 angeschlossen, die an der Fußinnenseite geführt sind.

Die dem ausgenommenen Bereich 19 (zwischen Ferse und Vorderfuß) nächsten drei Leiterbahnen 10 sind ebenfalls im wesentlichen an der Fußinnenseite mit leitfähigen Elementen 14 verbunden, während die vorderen Leiterbahnen 10 über leitfähige Elemente 14 angeschlossen sind, welche zunächst die Fußlängsachse im ausgenommenen Bereich 19 kreuzen und dann an der Fußaußenseite entlanglaufen.

Der Bereich der ersten drei Leiterbahnen vor dem ausgenommenen Bereich 19 ist hierbei über die Fußaußenseite hervorgezogen.

Auf der Rückseite der Trägerfolie 12 (Fig. 1) sind mit den Leiterbahnen 10 deckungsgleiche Abschirmflächen vorgesehen, die also in der Abbildung nicht sichtbar sind. In der Fig. 1 sind jedoch diejenigen Abschnitte der Abschirmflächen 17 zu sehen, welche über die Leiterbahnen 10 bzw. die leitfähigen Elemente 14 vorstehen, wobei eine nicht deckungsgleiche, sondern größerflächige Überdeckung bei allen leitfähigen Elementen 14 vorgesehen ist. Darüber hinaus sind an der Außenseite des Fußes, dem gegenüberliegend an der Innenseite des Fußes und im Bereich des Anschlußflachkabels 24 Lappen 21, 22 und 23 vorgesehen, welche über die dort liegenden, leitfähigen Elemente 14 weit hervorstehen.

Die in Fig. 2 gezeigte Anordnung unterscheidet sich von der nach Fig. 1 zunächst dadurch, daß dort die Leiterbahnen 11 im wesentlichen parallel zur Fußlängsachse angebracht sind. Weiterhin sind bei der in Fig. 2 gezeigten Anordnung die Leiterbahnen 11 im wesentlichen im Bereich 19 mit den dazugehörigen leitfähigen Elementen 15 verbunden, wobei der in Fig. 2 gezeigte Bereich 19 dem in Fig. 1 gezeigten Bereich 19 entspricht.

Bei der in Fig. 2 gezeigten Anordnung sind neben den gleich angeordneten Lappen 23 des Anschlußflachkabels 25 noch drei weitere Lappen 22, 32 und 33 vorgesehen, wobei der Lappen 22 die Fortsetzung des ausgenommenen Bereiches 19 darstellt und die Lappen 32 und 33 über die dort geführten leitfähigen Elemente 15 hervorstehen.

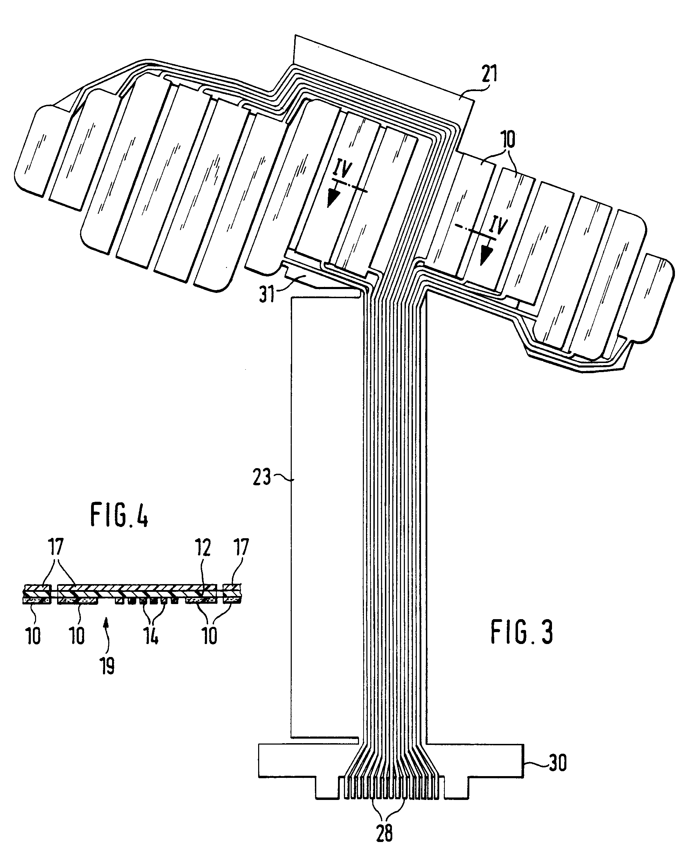

Nach dem Bedrucken der Trägerfolien wird im wesentlichen entlang der Außenumrisse der aufgedruckten Abschirmflächen ausgeschnitten. Dadurch ergibt sich der in den Fig. 3 und 4 erläuterte Aufbau, aus dem hervorgeht, daß zwischen den Leiterbahnen 10 die Folie ausgeschnitten wurde. Weiterhin geht aus Fig. 4 hervor, daß die Abschirmflächen 17 sowohl die Leiterbahnen 10 als auch die leitfähigen Elemente 14 von der Rückseite der Trägerfolie 12 her abdecken und somit gegen Störungen abschirmen können.

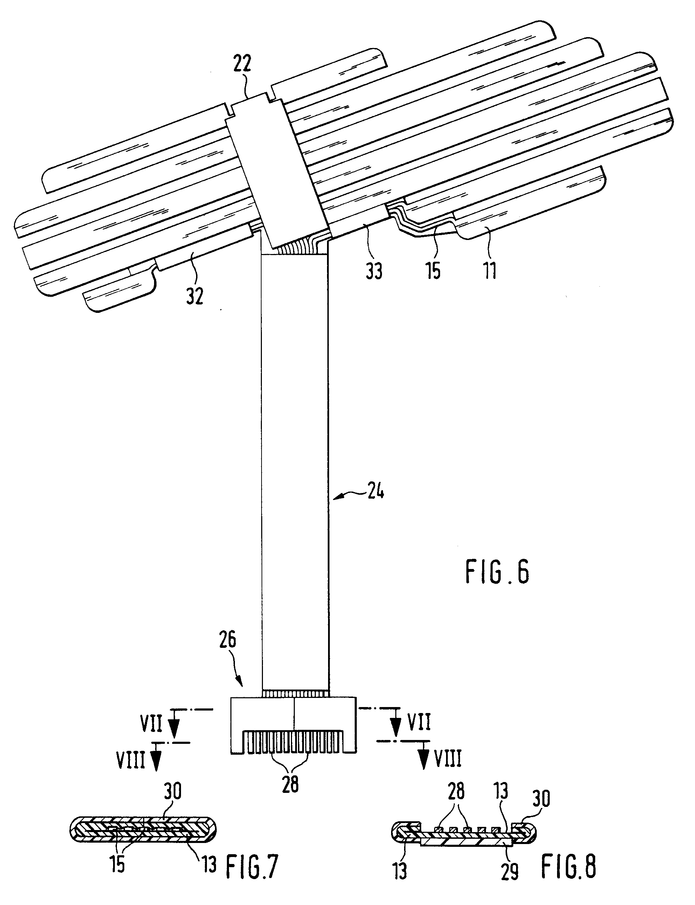

Nachdem man die "überschüssige" Trägerfolie fortgeschnitten hat, schlägt man die Lappen 21, 23 und 31 nach innen in Richtung auf die dort verlaufenden leitfähigen Elemente 14 um und klebt sie auf diese auf. Vorzugsweise erfolgt dieses Kleben über ein trägerloses Acrylklebeband, was sich nicht nur als besonders fest, sondern in Verbindung mit den hier verwendeten Werkstoffen und der genauen Bemessung der Klebeflächen als besonders günstig erwiesen hat. Auf die gleiche Weise wird auch mit der in Fig. 2 bereits erläuterten Folie verfahren, was dann zu der in Fig. 6 gezeigten Konfiguration führt.

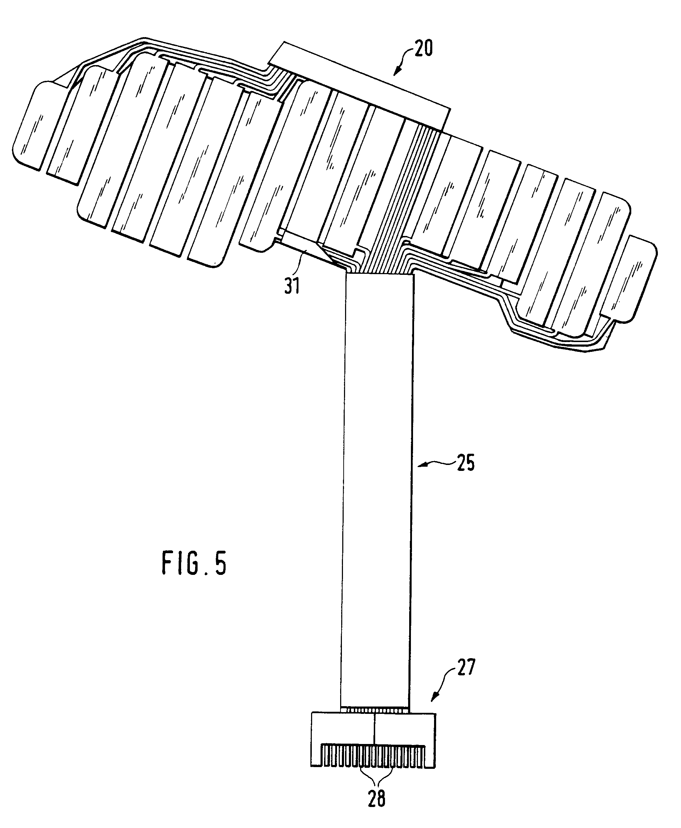

Bei der in Fig. 6 gezeigten Konfiguration fällt besonders noch auf, daß dort der ausgenommene Bereich 19 durch den eingeklappten und aufgeklebten Lappen 22 mit Abschirmfläche abgedeckt ist, was bei der in Fig. 5 gezeigten Fläche nicht der Fall ist.

Aus den Fig. 6 bis 8 geht noch ein weiteres wesentliches Detail hervor, das die Endbereiche 26/27 der Anschlußflachkabel 24 bzw. 25 betrifft. Dieser Endbereich ist nämlich derart ausgebildet, daß dort das Flachkabel 24 bzw. 25 mit jeweils einem Stecker verbindbar ist, über den die Anordnung an die hier nicht gezeigte Meßelektronik angeschlossen werden kann.

Aus der in Fig. 7 gezeigten Darstellung geht hervor, daß die Trägerfolie 13 mit den darauf befindlichen (aufgedruckten) leitfähigen Elementen 15 nach innen symmetrisch vom Rand her umgeschlagen wird, so daß die leitfähigen Elemente 15 zunächst von Trägerfolie 13 vollständig umhüllt sind. Nachdem aber die Trägerfolie 13 in diesem, im Bereich des Schnittes VII-VII liegenden Abschnitt durchgehend mit einer Abschirmfläche verbunden ist, die dort als Steckerabschirmung 30 dient, sind somit die leitfähigen Elemente 15 ringsum abgeschirmt. Diese Abschirmung geschieht also in einer extrem einfachen und dennoch wirksamen Weise.

Im äußersten Endabschnitt sind die leitfähigen Elemente 14 bzw. 15 zu Anschlußkontakten 28 auseinandergezogen, um dort einen Stecker anbringen zu können. Derartige Stecker sind handelsüblich und werden z.B. zum direkten Aufstecken auf Platinen verwendet. Die Anschlußkontakte 28 sind also ebenfalls (und gleichartig) aufgedruckt und unterscheiden sich von den Leiterbahnen 10/11 bzw. den leitfähigen Elementen 14/15 lediglich hinsichtlich Funktion und räumlicher Anordnung.

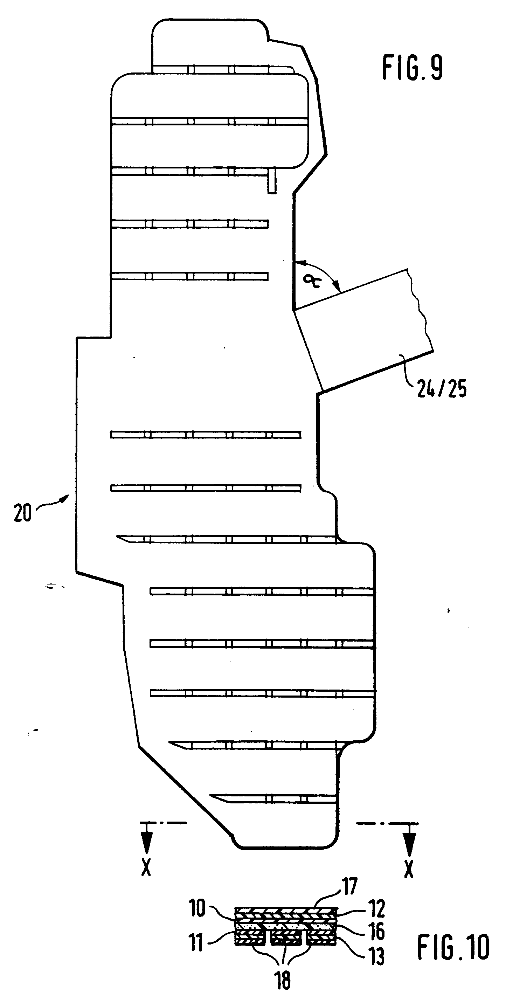

Wie in Fig. 8 gezeigt, ist im Bereich der Anschlußkontakte 28 auf der Rückseite der Trägerfolie 13 eine Versteifungsfläche 29 angebracht, die aus einer steiferen Kunstharzfolie besteht. Die Anbringung erfolgt hierbei ebenfalls wieder über Kleben, vorzugsweise mit trägerlosem Acrylklebeband. Durch diese Versteifung ist ein besonders sicheres und gefahrloses Einführen in den Stecker möglich.

Nachdem die in den Fig. 5 und 6 gezeigten und oben erläuterten Konfigurationen mit umgeklappten Klappen hergestellt sind, werden diese Anordnungen mit den Abschirmflächen 17, 18 nach außen auf eine entsprechend zugeschnittene Elastomer-Schicht 16 aufgeklebt, wie dies in den Fig. 9 und 10 dargestellt ist. Die elastomere Zwischenschicht 16 besteht vorzugsweise aus einem geschäumten Kunstharz mit geringer Hysterese.

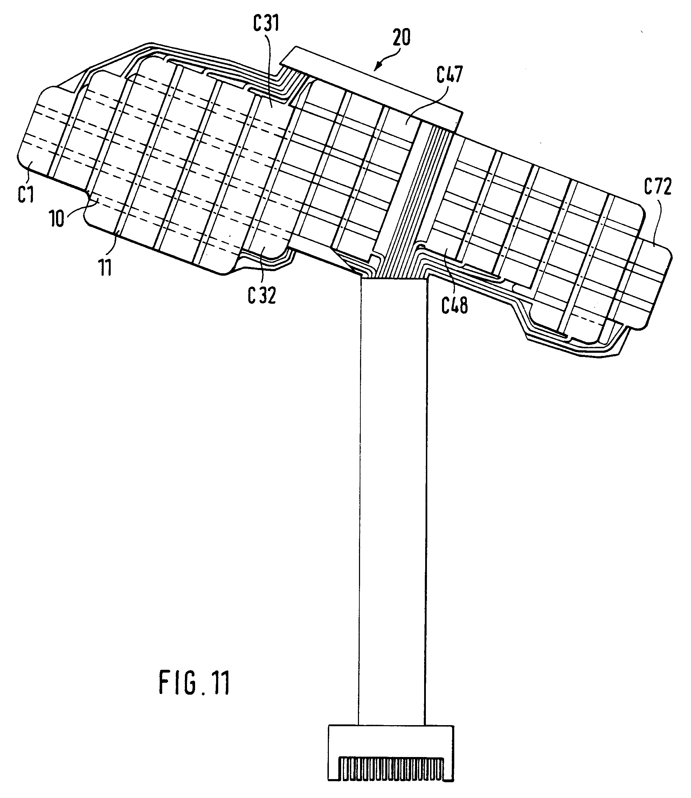

Wie dies aus den Fig. 10 und 11 hervorgeht, wird auf diese Weise die an sich bekannte Matrixanordnung geschaffen, bei der Kondensatorelemente C1 bis C72 gebildet sind. Jedes Kondensatorelement C1 bis C72 kann hierbei "angewählt" werden, wie dies aus den eingangs zitierten Druckschriften bekannt ist. Zu Fig. 11 ist hierbei noch zu bemerken, daß diese Abbildung lediglich zur Erläuterung der Kondensator-Anordnung dient, um den matrixförmigen Aufbau näher zu zeigen.

Wenn nun die in Fig. 9 gezeigte fertige "Meß-Einlegsohle" in den Schuh eines Probanden eingelegt werden soll, so werden die Anschlußflachkabel 24, 25 und der an der Fußaußenseite vorstehende Bereich 20 hochgeklappt. Hierbei ist der in Fig. 9 mit α bezeichnete Winkel, um den die Flachkabel 24/25 nach hinten, in Richtung Ferse geneigt sind, besonders vorteilhaft, da so die Meßkabel 24/25 knick- und zugfrei aus dem Schuh herausgeführt werden können.

Weiterhin wird dadurch, daß der Bereich 20 nach oben geklappt wird, eine Beeinflussung der Leitungskapazitäten der leitfähigen Elemente 14/15 in diesem Bereich ausgeschlossen, da dort nur geringe Kräfte beim Laufen und Gehen auftreten können.

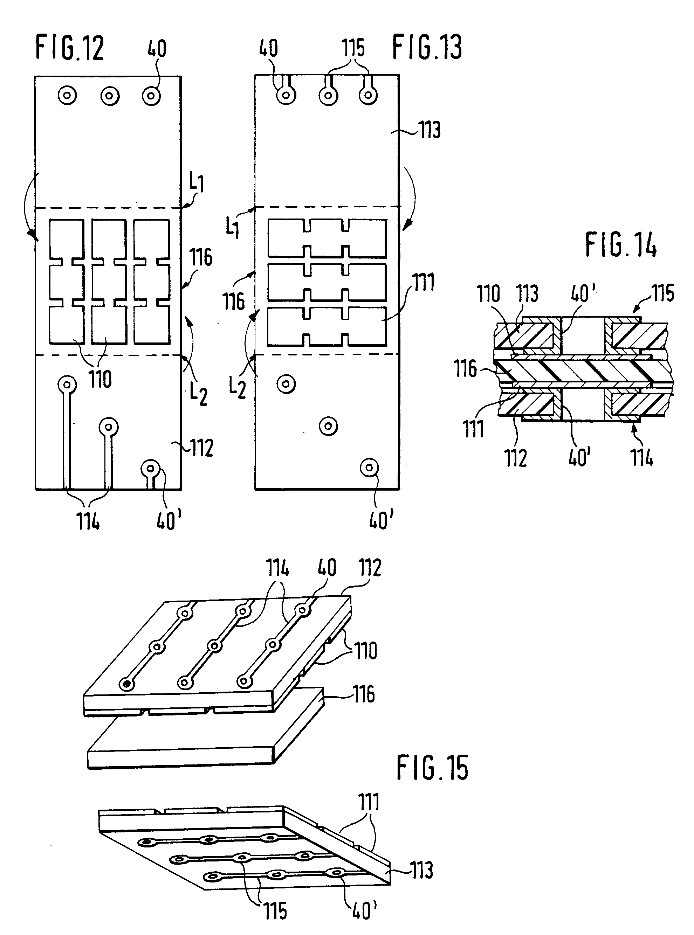

Im folgenden wird eine weitere bevorzugte Ausführungsform anhand der Fig. 12 bis 14 näher beschrieben. Hierbei zeigt die Fig. 12 die Draufsicht auf eine Seite, Fig. 13 auf die andere Seite einer bedruckten Elastomer-Folie, während Fig. 14 einen Teil-Schnitt durch eine aus einem Element nach den Fig. 12 und 13 zusammengebaute Vorrichtung zeigt.

Wie aus den Abbildungen hervorgeht, ist eine einzige Elastomerschicht vorgesehen, die in ihrem Mittelbereich das elastisch verformbare Dielektrikums 116 bildet, auf das die Kondensatorelemente 110, 111 (je auf einer Seite) aufgedruckt sind. Die Lage der Kondensatorelemente 110, 111 zueinander ist somit fest definiert. Die beiden Endbereiche der Folie können gegenüber dem Mittelbereich an den Knicklinien L1 und L2 abgeknickt werden, so daß jeweils ein Endbereich mit seiner Oberfläche auf eine Oberfläche des Mittelbereiches geklappt werden kann. Die Endbereiche bilden Trägerfolien 112, 113 für Leitungen 114, 115, die (wie dies später näher erklärt wird) den jeweiligen Kondensatorelementen 110 bzw. 111 zugeordnet sind.

Die Leitungen 114, 115 sind bis zum Rand der Elastomer-Folie geführt und enden innerhalb der Elastomer-Folie in Durch-Kontaktierungen 40, 40′, die einen Umgebungsrand auf der den Leitungen 114, 115 abgewandten Seiten der Elastomer-Folie aufweisen.

Klappt man die Fläche 112 so (in Fig. 13 aus der Zeichenebene heraus) auf die Fläche 116, daß die Durch-Kontaktierungen 40′ auf den Kondensatorelementen 111 zu liegen kommen und die Fläche 113 so (in Fig. 13 in die Zeichenebene hinein) auf die Fläche 116, daß die Durch-Kontaktierungen 40 auf den Kondensatorelementen 110 zu liegen kommen, ergibt sich eine dreischichtige Anordnung, wie sie in Fig. 14 im Schnitt dargestellt ist. Die Auflage der Durch-Kontaktierungen 40, 40′ auf den dazugehörigen Kondensatorelementen 110, 111 stellt bereits einen relativ sicheren Kontakt der Leitungen 114, 115 zu den Kondensatorelementen 110, 111 dar. Die Kontaktierung kann dadurch verbessert werden, daß vor dem Umklappen und Fixieren der aufeinandergelegten Schichten leitfähige Klebemassen auf die Umgebungsränder der Durch-Kontaktierungen 40, 40′ aufgebracht werden.

Bei einer weiteren, hier nicht in Abbildungen dargestellten bevorzugten Ausführungsform der Erfindung sind die drei Flächen von Anfang an nicht miteinander (an den Knicklinien L1, L2) verbunden.

Diese Ausführungsform eignet sich dann auch besonders gut zur Herstellung einer Meßvorrichtung in Form einer Einlegesohle wie oben beschrieben.

Bei einer weiteren, in Fig. 15 gezeigten bevorzugten Ausführungsform der Erfindung sind die Kondensatorelemente 110, 111 jeweils auf verschiedenen Trägerfolien 112, 113 aufgedruckt. Die jeweils dazugehörigen Leitungen 114, 115 sind auf der jeweils anderen Seite der Trägerfolien 112, 113 aufgedruckt und über Durch-Kontaktierungen 40, 40′ mit den dazugehörigen Kondensatorelementen 110, 111 verbunden. Diese beiden bedruckten Trägerfolien 112, 113 werden (wie beim eingangs beschriebenen Beispiel bereits gezeigt) mit den Kondensatorelementen 110, 111 nach innen weisend auf eine Elastomerschicht 116 aufgeklebt, welche das Dielektrikum bildet. Diese Anordnung kann auch in der aus der Herstellung von Platinen für gedruckte Schaltungen bekannten Multilayer-Technik ausgeführt werden.

Wie sich aus dem Vorstehenden ergibt, wird zum einen eine fertige Meßmatte bzw. Einlegesohle zum Messen der flächigen Verteilung von Druckkräften aufgezeigt, zum anderen ein Verfahren zur Herstellung einer solchen Vorrichtung. Ein wesentlicher Punkt ist hierbei der, daß durch die besondere Formgebung sowohl der Leiterbahn und deren Zuleitung über leitfähige Elemente als auch durch die besondere Formgebung der Abschirmflächen ein besonders (elektrisch) störungssicherer Aufbau erzielt wird, mit dem besonders exakte Messungen durchführbar sind. Die Herstellung ist einfach und kann kostengünstig in großen Serien erfolgen, da entgegen der bisherigen Auffassung der Fachleute keine besondere Formgebung der Leiterbahnen notwendig ist.

**Patentansprüche**

1. Vorrichtung zur Messung der flächigen Verteilung von Druckkräften, die im wesentlichen senkrecht zu einer verformbaren Meßfläche wirken,
mit einer Matrixanordnung von Kraftsensoren, die jeweils durch Kapazitäten (C1 bis C72) an Kreuzungspunkten zwischen Gruppen von Anordnungen von auf Träger (12, 13) aus Kunststoff aufgedruckten Kondensatorelementen (10, 11) gebildet sind, welche auf den einander gegenüberliegenden Oberflächen eines elastisch verformbaren Dielektrikums (16) zu befestigen sind und über Leitungen (14, 15) mit einer Auswertelektronik verbindbar sind, dadurch **gekennzeichnet,**
daß das elastisch verformbare Dielektrikum als flächiges Dielektrikum (16) aus Kunststoff ausgebildet ist, und daß die Kondensatorelemente (10, 11) zusammen mit den Leitungen (14, 15) auf Trägerfolien (12, 13) aus Kunststoff als Träger aufgedruckt sind.

2. Vorrichtung nach Anspruch 1,
dadurch **gekennzeichnet,**
daß die Träger (12, 13) aus einem Elastomer gefertigt sind.

3. Vorrichtung nach Anspruch 2,
dadurch **gekennzeichnet,**
daß das Elastomer eine PVC-Folie umfaßt.

4. Vorrichtung nach einem der vorhergehenden Ansprüche,
dadurch **gekennzeichnet,**
daß das Dielektrikum eine Schicht (16) aus einem vorzugsweise geschäumten Elastomer umfaßt, auf welcher die Kondensatorelemente (10, 11) mit nach außen weisendem Träger (12, 13) fixiert sind.

5. Vorrichtung nach einem der vorhergehenden Ansprüche,
dadurch **gekennzeichnet,**
daß die Träger (12, 13) auf das Dielektrikum (16) aufgeklebt sind, vorzugsweise mittels trägerlosem Acryl-Klebeband aufgeklebt sind.

6. Vorrichtung nach einem der vorhergehenden Ansprüche,
dadurch **gekennzeichnet,**
daß die Kondensatorelemente (10) auf mehreren Trägern (12, 13) mit jeweils dazwischenliegendem Dielektrikum (16) verteilt so angeordnet sind, daß sie übereinander projiziert gesehen eine im wesentlichen durchgehende Meßfläche bilden.

7. Vorrichtung zur Messung der flächigen Verteilung von Druckkräften, die im wesentlichen senkrecht zu einer verformbaren Meßfläche wirken, mit einer Matrixanordnung von Kraftsensoren, die jeweils durch Kapazitäten an Kreuzungspunkten zwischen zwei Gruppen von Anordnungen von auf einem Träger aufgedruckten Kondensatorelementen (110, 111) gebildet sind, welche auf den einander gegenüberliegenden Oberflächen eines elastisch verformbaren Dielektrikums (116) liegen und die über Leitungen (114, 115) mit einer Auswertelektronik verbindbar sind,
dadurch **gekennzeichnet,**
daß das elastisch verformbare Dielektrikum als flächiges Dielektrikum (112, 113, 116) aus Kunststoff ausgebildet ist, und daß die Kondensatorelemente (110, 111) zusammen mit den Leitungen (114, 115) auf das Dielektrikum als Träger aufgedruckt sind.

8. Vorrichtung nach Anspruch 7,
dadurch **gekennzeichnet,**
daß die Kondensatorelemente (110, 111) und die Leitungen (114, 115) auf zwei verschiedenen Seiten des Dielektrikums (112, 113) angebracht und über Durch-Kontaktierungen (40) in vorgegebener Weise miteinander verbunden sind, welche Innenauskleidungen von durchgehenden Ausnehmungen in dem Dielektrikum (112, 113) umfassen, wobei die Innenauskleidungen aus demselben Material wie die Kondensatorelemente (110, 111) bzw. die Leitungen (114, 115) bestehen.

9. Vorrichtung nach einem der vorhergehenden Ansprüche,
dadurch **gekennzeichnet,**
daß der Träger ein geschäumtes Elastomer umfaßt.

10. Vorrichtung nach einem der vorhergehenden Ansprüche,
dadurch **gekennzeichnet,**
daß die Leitungen (14, 15; 114, 115) und die Kondensatorelemente (10, 11; 110, 111) aus Silber-gefülltem Kunstharz bestehen.

11. Vorrichtung nach einem der vorhergehenden Ansprüche,
dadurch **gekennzeichnet,**
daß auf der den Kondensatorelementen (10, 11; 110, 111) gegenüberliegenden Seite Abschirmflächen (17, 18; 117, 118) aufgedruckt sind, welche die Kondensatorelemente (10, 11; 110, 111) gegebenenfalls auch die Leitungen (14, 15; 114, 115) überdecken.

12. Vorrichtung nach einem der vorhergehenden Ansprüche,
dadurch **gekennzeichnet,**
daß die Träger (12, 13; 112, 113) zwischen den Kondensatorelementen (10, 11; 110, 111) mindestens abschnittsweise durchtrennt sind.

13. Vorrichtung nach einem der vorhergehenden Ansprüche,
wobei die Meßfläche der Form einer Fuß-Auftrittsfläche angepaßt und in einen Schuh eines Probanden einlegbar ist,
dadurch **gekennzeichnet,**
daß die Kondensatorelemente (10, 11; 110, 111) reihenförmig angeordnet sind, wobei ein Satz von Kondensatorelementen (10, 110 quer zur Fußlängsachse, der andere Satz von Kondensatorelementen (11, 111) parallel zur Fußlängsachse verläuft.

14. Vorrichtung nach Anspruch 13,
dadurch **gekennzeichnet,**
daß die Leitungen (14, 15; 114, 115) im Bereich des Tuberosithas Ossis Navicularis zusammen mit einem Trägerfolienstreifen als Anschlußflachkabel (24, 25; 124, 125) aus dem Schuh herausführbar sind.

15. Vorrichtung nach einem der Ansprüche 13 oder 14,
dadurch **gekennzeichnet,**
daß diejenige Reihe von Kondensatorelementen (11, 111), die parallel zur Fußlängsachse verläuft und über die im wesentlichen gesamte Fußlänge reicht, im Bereich zwischen Ferse und Vorderfuß mit den Leitungen (15, 115) verbunden ist.

16. Vorrichtung nach Anspruch 14,
dadurch **gekennzeichnet,**
daß die Abschirmflächen (17, 18; 117, 118) im Bereich des Anschlußflachkabels (24, 25; 124, 125) vorgesehen und als ein über die Leitungen (14, 15; 114, 115) hinausragender Lappen (23) ausgeführt sind und die Leitungen (14, 15; 114, 115) abdeckend umklappbar auf diesen unter Zwischenlage des Trägers festklebbar sind.

17. Vorrichtung nach Anspruch 14,
dadurch **gekennzeichnet,**
daß die Enden (26, 27; 126, 127) der Anschlußflachkabel (24,> 25; 124, 125) derart mit Anschlußkontakten (28) versehen sind, daß ein handelsüblicher Direkt-Steckverbinder anbringbar ist.

18. Vorrichtung nach Anspruch 17,
dadurch **gekennzeichnet,**
daß auf den Träger im Bereich der Anschlußkontakte (28) eine Versteifungsfolie (29) aufgeklebt ist.

19. Vorrichtung nach einem der Ansprüche 14 bis 18,
dadurch **gekennzeichnet,**
daß die Anschlußflachkabel (24, 25; 124, 125) leicht nach hinten, in Richtung Ferse geneigt herausgeführt sind.

## Claims

1. A device for measuring the areal distribution of pressure forces acting substantially at right angles to a deformable surface to be measured, comprising a matrix arrangement of force sensors, which are each formed by capacitances (C1 to C72) at intersection points between groups of arrangements of capacitor elements (10, 11) printed onto carriers (12, 13) composed of synthetic resin, which capacitor elements (10, 11) are to be attached on the oppositely disposed surfaces of an elastically deformable dielectric (16) and can be connected via conductors (14, 15) to an analysis electronics unit, characterised in that the elastically deformable dielectric has the form of a planar dielectric (16) composed of synthetic resin and that the capacitor elements (10, 11) together with the conductors (14, 15) are printed onto carrier sheets (12, 13) composed of synthetic resin as carriers.

2. A device as claimed in Claim 1, characterised in that the carriers (12, 13) are produced from an elastomer.

3. A device as claimed in Claim 2, characterised in that the elastomer comprises a PVC sheet.

4. A device as claimed in one of the preceding claims, characterised in that the dielectric comprises a layer (16) of a preferably foamed elastomer, onto which the capacitor elements (10, 11), with an outwardly directed carrier (12, 13), are fixed.

5. A device as claimed in one of the preceding claims, characterised in that the carriers (12, 13) are glued onto the dielectric (16), preferably by means of unsupported acrylic adhesive tape.

6. A device as claimed in one of the preceding claims, characterised in that the capacitor elements (10) are distributed on a plurality of carriers (12, 13) with a respective dielectric (16) disposed therebetween, in such manner that when projected above one another they form a substantially continuous measurement surface.

7. A device for measuring the areal distribution of pressure forces acting substantially at right angles to a deformable surface to be measured, comprising a matrix arrangement of force sensors, which are each formed by capacitances at intersection points between two groups of arrangements of capacitor elements (110, 111) which are printed on a carrier and which are arranged on the oppositely disposed surfaces of an elastically deformable dielectric (116) and which can be connected via conductors (114, 115) to an analysis electronics unit, characterised in that the elastically deformable dielectric has the form of an areal dielectric (112, 113, 116) composed of synthetic resin and that the capacitor elements (110, 111) together with the conductors (114, 115) are printed onto the dielectric as carrier.

8. A device as claimed in Claim 7, characterised in that the capacitor elements (110, 111) and the conductors (114, 115) are arranged on two different sides of the dielectric (112, 113) and are connected to one another in a predetermined manner via through-contacts (40) which comprise inner linings of throughgoing openings in the dielectric (112, 113), where the inner linings are composed of the same material as the capacitor elements (110, 111) and the conductors (114, 115).

9. A device as claimed in one of the preceding claims, characterised in that the carrier comprises a foamed elastomer.

10. A device as claimed in one of the preceding claims, characterised in that the conductors (14, 15; 114, 115) and the capacitor elements (10, 11; 110, 111) are composed of silverfilled synthetic resin.

11. A device as claimed in one of the preceding claims, characterised in that screening surfaces (17, 18; 117, 118) are printed on the side opposite the capacitor elements (10, 11; 110, 111), which screening surfaces cover the capacitor elements (10, 11; 110, 111) and optionally also the conductors (14, 15; 114, 115).

12. A device as claimed in one of the preceding claims, characterised in that the carriers (12, 13; 112, 113) are interrupted, at least in sections, between the capacitor elements (10, 11; 110, 111).

13. A device as claimed in one of the preceding claims, wherein the surface to be measured is adapted to the shape of a foot tread area and can be inserted into a shoe of an individual to be tested, characterised in that the capacitor elements (10, 11; 110, 111) are arranged in rows, one set of capacitor elements (10, 110) extending transversely to the longitudinal axis of the foot and the other set of capacitor elements (11, 111) extending in parallel to the longitudinal axis of the foot.

14. A device as claimed in Claim 13, characterised in that the conductors (14, 15; 114, 115) can be led out of the shoe in the region of the tuberosithas ossis navicularis together with a strip of carrier sheeting as flat connection cable (24, 25; 124, 125).

15. A device as claimed in one of Claims 13 or 14, characterised in that the row of capacitor elements (11, 111) which extends in parallel to the longitudinal axis of the foot and runs substantially along the entire length of the foot is connected to the conductors (15, 115) in the region between the heel and the forefoot.

16. A device as claimed in Claim 14, characterised in that the screening surfaces (17, 18; 117, 118) are arranged in the region of the flat connection cable (24, 25; 124, 125) and comprise a flap (23), which projects beyond the conductors (14, 15; 114, 115), and can be folded over so as to cover the conductors (14, 15; 114, 115) in order to be glued onto said conductors with the carrier disposed therebetween.

17. A device as claimed in Claim 14, characterised in that the ends (26, 27; 126, 127) of the flat connection cables (24, 25; 124, 125) are provided with terminal contacts (28) in such manner that a commercially available direct plug connector can be applied.

18. A device as claimed in Claim 17, characterised in that a stiffening sheet (29) is glued onto the carrier in the region of the terminal contacts (28).

19. A device as claimed in one of Claims 14 to 18, characterised in that the flat connection cables (24, 25; 124, 125) are led out inclined slightly rearwards in the direction of the heel.

**Revendications**

1. Dispositif pour mesurer la répartition des efforts de compression dans une surface qui agissent essentiellement perpendiculairement à une surface de mesure déformable, comprenant un système matriciel de capteurs de force constitués respectivement par des capacités (C1 à C72) d'éléments de condensateur (10, 11) imprimés sur des supports (12, 13) en matière plastique aux croisements entre des groupes de systèmes, lesquels éléments de condensateur doivent être fixés sur les surfaces opposées d'un diélectrique (16) déformable de manière élastique et peuvent être reliés par l'intermédiaire de conducteurs (14, 15) à une électronique d'interprétation, **caractérisé en ce** le diélectrique déformable élastiquement est conformé en diélectrique plan (16) en matière plastique, et que les éléments de condensateur (10, 11) sont imprimés, conjointement avec les conducteurs (14, 15) sur des feuilles de support (12, 13) en matière plastique qui servent de substrat.

2. Dispositif selon la revendication 1, caractérisé en ce que les supports (12, 13) sont constitués par un élastomère.

3. Dispositif selon la revendication 2, caractérisé en ce que l'élastomère comprend une feuille de PVC.

4. Dispositif selon l'une des revendications précédentes, caractérisé en ce que le diélectrique comprend une couche (16) d'un élastomère expansé sur laquelle les éléments de condensateur (10, 11) sont fixés avec le support (12, 13) dirigé vers l'extérieur.

5. Dispositif selon l'une des revendications précédentes, caractérisé en ce que les supports (12, 13) sont collés sur le diélectrique (16), de préférence au moyen d'un ruban adhésif acrylique sans support.

6. Dispositif selon l'une des revendications précédentes, caractérisé en ce que les éléments de condensateur (10) sont répartis sur plusieurs supports (12, 13) avec à chaque fois le diélectrique (16) interposé, de sorte qu'ils forment, vu en projection superposée, une surface de mesure sensiblement continue.

7. Dispositif pour mesurer la répartition des efforts de compression dans une surface qui agissent essentiellement perpendiculairement à une surface de mesure déformable, comprenant un système matriciel de capteurs de force constitués respectivement par des capacités d'éléments de condensateur (110, 111) imprimés sur un support aux croisements entre deux groupes de systèmes, lesquels éléments de condensateur se situent sur les surfaces opposées d'un diélectrique (116) déformable de manière élastique et peuvent être reliés par l'intermédiaire de conducteurs (114, 115) à une électronique d'interprétation, caractérisé en ce que le diélectrique déformable élastiquement est conformé en diélectrique plan (112, 113, 116) en matière plastique, et que les éléments de condensateur (110, 111) sont imprimés, conjointement avec les conducteurs (114, 115), sur le diélectrique qui sert de substrat.

8. Dispositif selon la revendication 7, caractérisé en ce que les éléments de condensateur (110, 111) et les conducteurs (114, 115) sont disposés sur deux côtés différents du diélectrique (112, 113) et reliés entre eux de manière prédéterminée par l'intermédiaire de contacts traversants (40) lesquels comprennent des revêtements intérieurs d'évidements continus à l'intérieur du diélectrique (112, 113), les revêtements intérieurs étant constitués du même matériau que les éléments de condensateur (110, 111) et respectivement les conducteurs (114, 115).

9. Dispositif selon l'une des revendications précédentes, caractérisé en ce que le support comprend un élastomère expansé.

10. Dispositif selon l'une des revendications précédentes, caractérisé en ce que les conducteurs (14, 15; 114, 115) et les éléments de condensateur (10, 11; 110, 111) sont constitués de résine synthétique chargée d'argent.

11. Dispositif selon l'une des revendications précédentes, caractérisé en ce que sur la face opposée aux éléments de condensateur (10, 11; 110, 111) sont imprimées des surfaces de blindage (17, 18; 117, 118) qui recouvrent les éléments de condensateur (10, 11; 110, 111) et éventuellement aussi les conducteurs (14, 15; 114, 115)

12. Dispositif selon l'une des revendications précédentes, caractérisé en ce que les supports (12, 13; 112, 113) sont coupés au moins par sections entre les éléments de condensateur (10, 11; 110, 111).

13. Dispositif selon l'une des revendications précédentes, la surface de mesure étant adaptée à la forme d'une surface de pose de pied et pouvant être insérée dans la chaussure d'un essayeur, caractérisé en ce que les éléments de condensateur (10, 11. 110, 111) sont disposés en rangées; un jeu d'éléments de condensateur (10, 110) s'étendant transversalement à l'axe longitudinal du pied et l'autre jeu d'éléments de condensateur (11, 111) parallèlement à l'axe longitudinal du pied.

14. Dispositif selon la revendication 13, caractérisé en ce que les conducteurs (14, 15; 114, 115) peuvent être sortis de la chaussure, conjointement avec une bande de feuille de support comme câble plat de raccordement (24, 25; 124, 125), dans la région de la tubérosité des os naviculaires.

15. Dispositif selon l'une des revendications 13 ou 14, caractérisé en ce que la rangée d'éléments de condensateur (11, 111) qui est orientée parallèlement à l'axe longitudinal du pied et s'étend sensiblement sur toute la longueur du pied, est reliée aux conducteurs (15, 115) dans la région entre le talon et l'avant du pied.

16. Dispositif selon la revendication 14, caractérisé en ce que les surfaces de blindage (17, 18; 117, 118) sont prévues dans la région du câble plat de raccordement (24, 25; 124, 125) et réalisées sous la forme d'une patte (23) dépassant des conducteurs (14, 15; 114, 115), et qu'elles peuvent être rabattues sur les conducteurs (14, 15; 114, 115) de façon à les recouvrir et collées sur ceux-ci avec interposition du support.

17. Dispositif selon la revendication 14, caractérisé en ce que les extrémités (26, 27; 126, 127) des câbles plats de raccordement (24, 25; 124, 125) sont munies de contacts de raccordement (28) de façon à permettre la mise en place d'un connecteur direct de type commercial.

18. Dispositif selon la revendication 17, caractérisé en ce qu'une feuille de renforcement (29) est collée sur le support dans la région des contacts de raccordement (28).

19. Dispositif selon l'une des revendications 14 à 18, caractérisé en ce que les câbles plats de raccordement (24, 25; 124, 125) sont sortis de manière légèrement inclinée vers l'arrière en direction du talon.

FIG. 2

FIG. 1

FIG.4

FIG.3

FIG.5

EP 0 279 361 B1

22

32

33    15    11

24

FIG.6

26

VII          VII

VIII        VIII

30                          28    13    30
28

15    13  FIG.7        13        29  FIG.8

14

FIG.9

α

24/25

20

X          X

FIG.10

17
10      12
16
11      13
18

FIG.11

FIG.12

FIG.13

FIG.14

FIG.15